Europäisches Patentamt

European Patent Office

Office européen des brevets

(11) Veröffentlichungsnummer : **0 143 107**
**B1**

# (12) EUROPÄISCHE PATENTSCHRIFT

(45) Veröffentlichungstag der Patentschrift :
**17.08.88**

(51) Int. Cl.⁴ : **G 01 N 33/53, G 01 N 33/02,**
**G 01 N 33/04, G 01 N 33/14**

(21) Anmeldenummer : **83110080.5**

(22) Anmeldetag : **26.10.83**

(54) **Neues Verfahren zum Virus-Nachweis in Reinkulturen mikroskopischer Kleinlebewesen sowie in Lebensmitteln und in technologischen Produkten.**

(43) Veröffentlichungstag der Anmeldung :
**05.06.85 Patentblatt 85/23**

(45) Bekanntmachung des Hinweises auf die Patenterteilung : **17.08.88 Patentblatt 88/33**

(84) Benannte Vertragsstaaten :
**AT BE CH DE FR GB IT LI LU NL SE**

(56) Entgegenhaltungen :
**EP-A- 0 038 150**
**WO-A-83 /005 05**
**FR-A- 2 439 233**

(73) Patentinhaber : **Lembke, Jürgen, Dr.**
**Eutiner Strasse 11**
**D-2420 Eutin-Sielbeck (DE)**

(72) Erfinder : **Lembke, Jürgen, Dr.**
**Eutiner Strasse 11**
**D-2420 Eutin-Sielbeck (DE)**

(74) Vertreter : **Tönnies, Jan G., Dipl.-Ing.**
**Niemannsweg 133**
**D-2300 Kiel 1 (DE)**

**Beschreibung**

Die Erfindung betrifft ein Verfahren zum Nachweis von Bakteriophagen durch Zugabe phagenspezifischer Antikörper.

Mikroorganismen werden infolge ihres Fermentreichtums und ihrer hohen Vermehrungsgeschwindigkeit zu vielfältigen medizinischen und technologischen Zwecken, z. B. für die Biosynthese von Nährstoffen und Vitaminen sowie für organisch-chemische Synthesen benutzt. Fast ausnahmslos verwendet man hierbei Reinkulturen mit spezifischen Fähigkeiten, die von natürlichen Standorten isoliert werden.

Nach den Regeln der Kunst ist die Herstellung von Reinkulturen, die sich von einer Zelle herleiten, möglich. Häufig wird beobachtet, daß eine Reinkultur ihre biochemischen Merkmale verändert und dadurch für vorgesehene Zwecke unbrauchbar wird. Eine solche Erscheinung liegt beispielsweise vor, wenn der für die Rahmsäuerung benutzte Keim Streptococcus diacetylactis unerwünschte Geschmacksstoffe entstehen läßt, wenn der für die Käsereifung erforderliche Keim Lactobacillus helveticus sein Eiweißabbau-Vermögen verliert oder wenn Propionsäurebakterien Lactat nicht mehr abzubauen vermögen.

Solche Änderungen der biochemischen Leistungen sind selten auf eine Selektion genetischer Varianten zurückzuführen sondern vielmehr auf eine Infektion mit Bakterienviren, sogenannter Bakteriophagen, die entweder die erwünschten Reinkulturen zerstören oder so verändern, daß sie technologisch unbrauchbar werden.

Es ist davon auszugehen, daß Bakteriophagen für fast alle Bakterien vorhanden sind und durch diese ungehemmt reproduziert werden können. Bakteriophagen zerstören normalerweise die Bakterienzelle, sie können aber auch als sogenannte temperente Phagen mehrfach bei der Zellteilung unerkannt weitergegeben werden. Hierdurch können große praktische Schäden bei der Verwendung von Reinkulturen zunächst unerkannt bleiben ; phagenanfällige Bakterienstämme sind daher nachzuweisen, auszusondern und durch phagenresistente Bakterienstämme zu ersetzen.

Ausgehend von dem erstmals in : Clark, M.F. und A.N. Adams : J. Gen. Virol. 34, 475, (1977) vorgeschlagenem, allgemein als ELISA-Test bezeichneten Verfahren werden in der FR-A-24 39 233, der WO-A-83 00 505 und der EP-A-00 38 150 Verfahren zum Nachweis von Bakteriophagen durch Zugabe phagenspezifischer Antikörper angegeben. Diese Verfahren sind jedoch nicht immer ausreichend zuverlässig, da bei ihnen die Nachweisschwelle relativ hoch liegt.

Der Erfindung liegt daher die Aufgabe zugrunde, ein Verfahren zu schaffen, mittels dessen schon relativ geringe Mengen von Bakteriophagen schnell und zuverlässig nachgewiesen werden können.

Erfindungsgemäß wird diese Aufgabe gelöst durch Immobilisieren von Bakterienwirtzellen ohne Schädigung ihrer phagenspezifischen Rezeptoren und Inkubieren der immobilisierten Bakterienwirtzellen, wobei die immobilisierten Bakterienwirtzellen zum Aussondern phagensensitiver Bakterienstämme zusammen mit der Probe und zum Selektieren phagenresistenter Bakterienstämme zusammen mit definierten Bakteriophagen inkubiert werden. Vorzugsweise werden die immobilisierten Bakterienwirtzellen vor dem Inkubieren fixiert.

Eine Vermehrung etwa vorhandener Phagen und damit ein zuverlässigeres Ansprechen auf die zugegebenen Antikörper wird durch Zugabe von reproduktionsfähigen Bakterienwirtzellen zu dem immobilisierten Bakterienwirtzellen vor dem Inkubieren bewirkt.

Das Immobilisieren der Bakterienwirtzellen kann mittels Waschen der Bakterienwirtzellen in Salzlösung und Ein bringen der Bakterienwirtzellensuspension in ein Reaktionsgefäß und Inkubieren der Bakterienwirtzellensuspension, mittels Einbringen der Bakterienwirtzellen in ein mit einer Nährlösung versehenes Reaktionsgefäß und Bebrüten oder durch andere geeignete Verfahren durchgeführt werden. Das Fixieren der immobilisierten Bakterienwirtzellen erfolgt vorzugsweise mittels kurzzeitigem Aufbringen kalter Glutaraldehydlösung schwacher Konzentration.

Das erfindungsgemäße Verfahren soll an einem Ausführungsbeispiel erläutert werden, bei dem eine Probe auf Anwesenheit von Bakteriophagen untersucht werden soll. Dabei wird Bezug genommen auf zwei Abbildungen. Dabei zeigt :

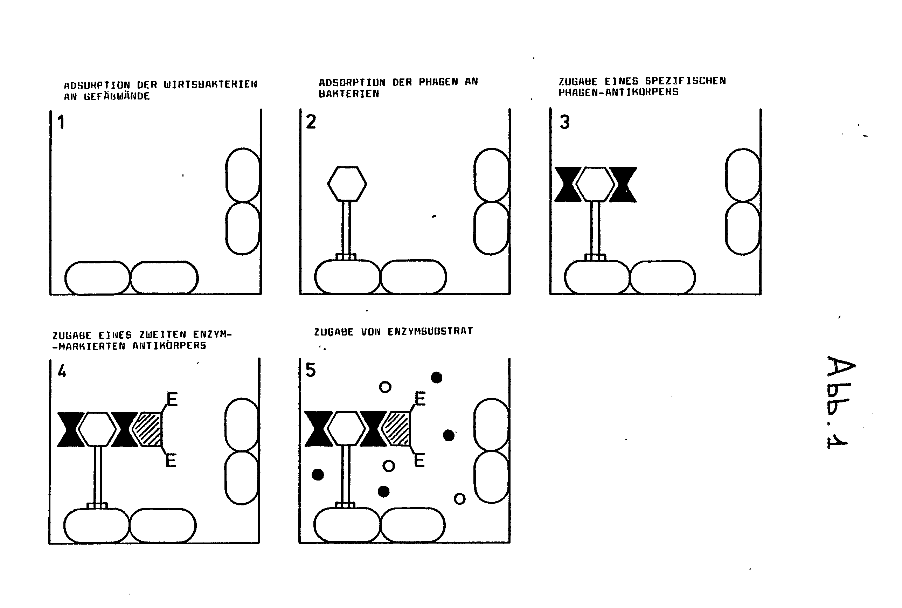

Abb. 1 ein Schema eines modifizierten ELISA-Tests zum Bakteriophagennachweis, bei dem vorhandene Phagen von Rezeptoren auf den Oberflächen immobilisierter Bakterien adsorbiert werden und der Bakteriophagennachweis mittels eines zweiten Enzym-markierten Antikörpers erfolgt, und

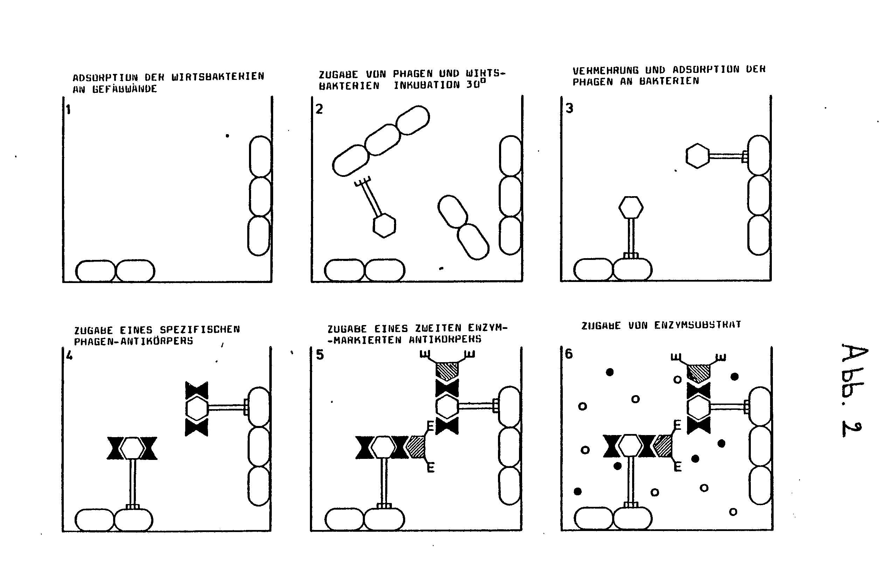

Abb. 2 ein Schema eines verbesserten modifizierten ELISA-Test zum Bakteriophagennachweis, bei dem in die mit immobilisierten Bakterien beschichteten Tröge stoffwechselnde Bakterien der gleichen Spezies gefüllt werden, vorhandene Phagen von diesem vermehrt an die Rezeptoren der immobilisierten Bakterien überwunden werden und der Bakteriophagennachweis der mittels eines zweiten Enzym-markierten Antikörpers erfolgt.

Aus einer käuflichen Reinkultur für die Bereitung von Sauerrahm wird unter sterilen Kautelen eine Probe gezogen und mit sterilem Wasser in Zehnerpotenzen fallend soweit verdünnt, daß bei Übertragung der letzten Verdünnungsstufe auf Nähragar-Platten die vorhandenen Reinkulturen durch Koloniewachstum in die Erscheinung treten. Mit der Impföse entnimmt man von den Kolonien jeweils ca. 0,1-1 mg Material und überführt dieses in eine bekannte Menge sterilen Wassers, die danach schwach getrübt erscheint. 10-20 ul dieser Suspension überträgt man z. B. in die mit ca. 300 ul einer Nährlösung gefüllten Tröge einer Mikrotiter-Platte und bebrütet diese 20 Stunden. Danach wird die Nährlösung aus den Vertiefungen entfernt, wobei die an den Trogwänden haftenden kompletten Bakterien zurückbleiben, die nunmehr durch Einwirkung von Glutaraldehyd (0,1 - 0,2 % in Puffer-, pH 6-7) fixiert und an die Wandung gebunden werden. Nach Entfernen des Glutaraldehyds müssen die Gefäße bzw. Mikrotiter-Platten mehrfach gründlich gewaschen werden (ca. 3-5 x a 10 Stunden). Die Spüllösung enthält außer Puffer 0,1 % Glycin, 0,2 % Tween 20 (eingetragenes Warenzeichen), 0,5 % Serumalbumin vom Rind und 2 % Kälberserum. Anschließend werden die auf das Vorhandensein von Bakteriophagen zu untersuchenden Proben in die in die Tröge eingebracht und inkubiert. Ist der für den immobilisierten Bakterienstamm « passende » Bakterienphage in der Probe zugegen, so wird er von den nicht-denaturierten Rezeptoren der Bakterienoberfläche gebunden. Nach 1-12 Stunden Inkubation ist eine vollständige Bindung der vorhandenen Phagen eingetreten, so daß erneut mit Spüllösung gewaschen werden kann. Nunmehr wird ein spezifischer Phagen-Antikörper in das System eingebracht und ungebundene Antikörper nach ein bis mehrstündiger Inkubation mit der beschriebenen Pufferlösung durch mehrfaches Waschen entfernt. Danach wird ein spezifischer Enzym-markierter Antikörper, der gegen den Phagen-Antikörper (indirekter Nachweis) gerichtet ist, dem System zugeführt. Nach erfolgter Bindung wird erneut gewaschen und Enzymsubstrat (z. B. o-Phenylendiamin für Peroxidase) zugegeben ; nach 20 Minuten ist die Gegenwart von Phagen an der eingetretenen Farbreaktion deutlich abzulesen.

Eine entscheidende Verbesserung der Erfassung kleinster Phagenmengen ist dadurch möglich, daß in das vorstehend beschriebene Nachweissystem mit immobilisierten Bakterien stoffwechselnde Bakterienzellen der gleichen Spezies vor dem Inkubieren eingebracht werden, die nach kurzer Inkubation (1-10 Stunden) eine extreme Bakteriophagenvermehrung ergeben und damit die Erfassung von 1-5 Phagenteilchen/ml ermöglicht. Die Sichtbarmachung erfolgt wie vorstehend beschrieben durch direkten oder indirekten Nachweis mit Antikörper-Enzym.

Zum Finden phagenresistenter Bakterienstämme wird das beispielhaft angegebene Vorgehen derart modifiziert, daß den immobilisierten Bakterienstämmen vor dem Inkubieren nicht die Probe sondern definierte Bakteriophagen zugegeben werden. Dabei werden die zugegebenen definierten Bakteriophagen von phagensensitiven Bakterienstämmen gebunden und können durch Zugabe phagenspezifischer Antikörper nachgewiesen werden.

## Patentansprüche

1. Verfahren zum Nachweis von Bakteriophagen durch Zugabe phagenspezifischer Antikörper, um phagensensitive Bakterienstämme in einer Probe auszusondern, gekennzeichnet durch Immobilisieren von Bakterienwirtzellen ohne Schädigung ihrer phagenspezifischen Rezeptoren und Inkubieren der immobilisierten Bakterienzellen zusammen mit der Probe vor Zugabe der phagenspezifischen Antikörper.

2. Verfahren zum Nachweis von Bakteriophagen durch Zugabe phagenspezifischer Antikörper, um phagenresistente Bakterienstämme auszuwählen, gekennzeichnet durch Immobilisieren von Bakterienwirtzellen ohne Schädigung ihrer phagenspezifischen Rezeptoren und Inkubieren der immobilisierten Bakterienzellen zusammen mit definierten Bakteriophagen vor Zugabe der phagenspezifischen Antikörper.

3. Verfahren nach Anspruch 1 oder Anspruch 2, gekennzeichnet durch Fixieren der immobilisierten Bakterienwirtzellen vor dem Inkubieren.

4. Verfahren nach einem der vorangehenden Ansprüche, gekennzeichnet durch Zugabe von reproduktionsfähigen Bakterienwirtzellen zu den immobilisierten Bakterienwirtzellen vor dem Inkubieren.

5. Verfahren nach einem der vorangehenden Ansprüche, gekennzeichnet durch Immobilisieren der Bakterienwirtzellen mittels Waschen der Bakterienwirtzellen in Salzlösung, Einbringen der Bakterienwirtzellensuspension in ein Reaktionsgefäß und Inkubieren der Bakterienwirtzellensuspension.

6. Verfahren nach einem der Ansprüche 1 bis 4, gekennzeichnet durch Immobilisieren der Bakterienwirtzellen mittels Einbringen der Bakterienwirtzellen in ein mit einer Nährlösung versehenes Reaktionsgefäß und Bebrüten dieser Suspension.

7. Verfahren nach einem der Ansprüche 4 bis 6, gekennzeichnet durch Fixieren der immobilisierten Bakterienwirtzellen mittels kurzzeitigen Aufbringens kalter Glutaraldehydlösung schwacher Konzentration.

## Claims

1. Method for detecting bacteriophages by adding phagespecific antibodies so as to sort out phage-sensitive bacterial strains in a sample, characterized by the immobilization of bacterial host cells without damaging their phage-specific receptors and by the incubation of the immobilized bacterial cells together with the sample before adding the phage-specific antibodies.

2. Method for detecting bacteriophages by adding phagespecific antibodies so as to separate out phage-resistant bacterial strains, characterized by the immobilization of bacterial host cells without damaging their phage-specific receptors and by the incubation of the bacterial cells together with defined bacteriophages before adding the phage-specific antibodies.

3. Method according to Claim 1 or Claim 2, characterized by the fixation of immobilized bacterial host cells before incubation.

4. Method according to one of the preceding claims, characterized by the addition of bacterial host cells capable of reproduction to the immobilized bacterial host cells before incubation.

5. Method according to one of the preceding claims, characterized by the immobilization of bacterial host cells by means of washing the bacterial host cells in a salt solution, placing the bacterial host cell suspension in a reaction vessel and incubating this suspension.

6. Method according to claims 1 to 4, characterized by the immobilization of bacterial host cells by means of placing the bacterial host cells into a reaction vessel which contains a breeding solution and incubating this suspension.

7. Method according to claims 4 to 6, characterized by the fixation of the immobilized bacterial host cells by means of briefly introducing a diluted glutaraldehyde solution.

**Revendications**

1. Méthode pour la détection de bactériophages par la donnée d'anticorps spécifiques pour la phage, pour éliminer des classes bactérielles réceptives à la phage dans un échantillon, caractérisées par l'immobilisation de cellules résidantes bactérielles sans détérioration de cellules résidantes bactérielles sans détérioration de leurs récepteurs spécifiques à la phage et de l'incubation des cellules bactérielles immobilisées ensemble avec l'échantillonage avant la donnée d'anticorps spécifiques à la phage.

2. Méthode pour la détection de bactériophages par la donnée d'anticorps spécifiques à la phage pour choisir des classes bactérielles résistantes à la phage caractérisées par l'immobilisation de cellules bactérielles résidantes sans détérioration de leurs récepteurs spécifiques à la phage et de l'incubation des cellules bactérielles en rapport avec les bactériophages définies avant la donnée des anticorps spécifiques à la phage.

3. Méthode selon la revendication 1 ou la revendication 2 caractérisée par la fixation des cellules résidantes bactérielles immobilisées avant l'incubation.

4. Méthode selon l'une des revendications précédentes, caractérisée par la donnée de cellules résidantes bactérielles susceptibles d'être reproduites pour les cellules résidantes bactérielles immobilisées avant l'incubation.

5. Méthode selon l'une des revendications précédentes caractérisée à travers l'immobilisation de cellules résidantes bactérielles à l'aide du lavage des cellules résidantes bactérielles dans une solution salée, mise de la suspension des bactéries résidantes cellulaires dans un récipient à réaction et l'incubation de la suspension des bactéries résidantes cellulaires en suspension.

6. Méthode selon l'une des revendications 1 jusqu'à 4 à travers l'immobilisation des cellules résidantes bactérielles à l'aide de la mise des cellules résidantes bactérielles dans un récipient à réaction rempli d'une solution nourrissante et de l'incubation de cette suspension.

7. Méthode selon l'une des revendications 4 jusqu'à 6 caractérisée à travers la fixation des cellules résidantes bactérielles immobilisées à l'aide de l'apport momentané d'une solution froide glutaraldehyde de faible concentration.

ADSORPTION DER WIRTSBAKTERIEN
AN GEFÄßWÄNDE

1

ADSORPTION DER PHAGEN AN
BAKTERIEN

2

ZUGABE EINES SPEZIFISCHEN
PHAGEN-ANTIKÖRPERS

3

ZUGABE EINES ZWEITEN ENZYM-
-MARKIERTEN ANTIKÖRPERS

4

E

E

ZUGABE VON ENZYMSUBSTRAT

5

E

E

0 143 107

Abb. 1

ADSORPTION DER WIRTSBAKTERIEN AN GEFÄßWÄNDE

1

ZUGABE VON PHAGEN UND WIRTS-BAKTERIEN  INKUBATION 30°

2

VERMEHRUNG UND ADSORPTION DER PHAGEN AN BAKTERIEN

3

ZUGABE EINES SPEZIFISCHEN PHAGEN-ANTIKÖRPERS

4

ZUGABE EINES ZWEITEN ENZYM--MARKIERTEN ANTIKÖRPERS

5

ZUGABE VON ENZYMSUBSTRAT

6

Abb. 2

0 143 107